(19) Europäisches Patentamt
European Patent Office
Office européen des brevets

(11) Publication number: **0 014 059**
**B1**

(12) **EUROPEAN PATENT SPECIFICATION**

(45) Date of publication of patent specification: **14.03.84**

(51) Int. Cl.³: **C 01 B 33/28**, C 01 B 33/20, B 01 J 29/28, B 01 J 29/04

(21) Application number: **80300122.1**

(22) Date of filing: **15.01.80**

(54) **New form of zeolite ZSM-11, preparation thereof and catalytic conversion therewith.**

(30) Priority: **15.01.79 US 3143**
**15.01.79 US 3145**

(43) Date of publication of application:
**06.08.80 Bulletin 80/16**

(45) Publication of the grant of the patent:
**14.03.84 Bulletin 84/11**

(84) Designated Contracting States:
**BE DE FR GB IT NL**

(56) References cited:
**DE - A - 2 755 770**
**FR - A - 2 386 483**
**GB - A - 1 161 974**
**US - A - 3 709 979**
**US - A - 4 061 724**
**US - A - 4 108 881**
**US - A - 4 112 056**

**NATURE, vol. 280, no. 5724, August 23, 1979, London, GB D.M. BIBBY et al. "Silicalite-2, a silica analogue of the alumino-silicate zeolite ZSM-11", pages 664-5.**

**The file contains technical information submitted after the application was filed and not included in this specification**

(73) Proprietor: **MOBIL OIL CORPORATION**
**150 East 42nd Street**
**New York New York 10017 (US)**

(72) Inventor: **Rubin, Mae Koenig**
**50 Belmont Avenue Bala Cynwyd**
**Pennsylvania 19004, Montgomery (US)**
Inventor: **Plank, Charles Joseph**
**522 Delaware Street**
**Woodbury New Jersey 08096, Gloucester (US)**
Inventor: **Rosinsky, Edward Joseph**
**Route No. 1 Box 325a**
**Pedricktown New Jersey 08067, Salem (US)**
Inventor: **Dwyer, Francis Gerard**
**1128 Talleyrand Road**
**West Chester Pennsylvania 19380, Chester (US)**

(74) Representative: **Cooper, John Anthony et al.**
**CARPMAELS & RANSFORD 43 Bloomsbury Square**
**London WC1A 2RA (GB)**

**EP 0 014 059 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# New form of zeolite ZSM-11, preparation thereof and catalytic conversion therewith

This invention relates to a new form of the zeolite ZSM/11, to a method for its preparation and to its use in catalytic conversion of organic compounds.

Prior art techniques have resulted in the formation of a great variety of synthetic aluminosilicates. These aluminosilicates have come to be designated by letter or other convenient symbols, as illustrated by zeolite A (U.S. Patent 2,882,243), zeolite X (U.S. Patent 2,882,244), zeolite Y (U.S. Patent 3,130,007), zeolite ZK-5 (U.S. Patent 3,247,195), zeolite ZK-4 (U.S. Patent 3,314,752), zeolite ZSM-5 (U.S. Patent 3,702,886), and zeolite ZSM-11 (U.S. Patent 3,709,979), merely to name a few.

The $SiO_2/Al_2O_3$ ratio of a given zeolite is often variable. For example, zeolite X can be synthesized with $SiO_2/Al_2O_3$ ratio of from 2 to 3; zeolite Y, from 3 to about 6. In some zeolites the upper limit of $SiO_2/Al_2O_3$ ratio is unbounded. ZSM-5 is one such example wherein $SiO_2/Al_2O_3$ ratio is at least five. U.S. Patent 3,941,871 discloses a crystalline metal organosilicate essentially free of aluminium and exhibiting an X-ray diffraction pattern characteristic of ZSM-5 type aluminosilicates. U.S. Patents 4,061,724, 4,073,865 and 4,104,294 describe microporous, crystalline silicas or organosilicates. The present invention is directed to a form of zeolite ZSM-11 having silica to alumina ratio higher than previously disclosed and indeed attaining an effectively alumina-free condition.

FR—A—2,386,483 discloses crystalline zeolites having a silica/alumina ratio between 10 and 3000, prepared from a silica-rich mixture which contains no alkali or alkaline earth cations. US—A—4,112,056 discloses preparation of ZSM-5 by addition of a source of aluminium ions to a silica-rich reaction mixture also containing a source of alkali metal oxide, organic nitrogen containing oxide and water.

The composition of the new form of ZSM-11 can be identified, in terms of mole ratios of oxides as follows:

$$(0\text{—}10)M_{2/n}O:(0\text{—}0.5)Al_2O_3:(100SiO_2$$

where M is at least one cation having a valence n. In the as synthesized form, the zeolite has a formula, after dehydration, in terms of moles of oxides, per 100 moles of silica, as follows:

$$(0\text{—}10)R_2O:(0\text{—}10)M_{2/n}O:(0\text{—}0.5)Al_2O_3:(100)SiO_2$$

wherein M is an alkali or alkaline earth metal, $R_2O$ is an organic compound of Group 5A element of the Periodic Table, preferably nitrogen or phosphorous, containing at least one alkyl or aryl group having between 1 and 7 carbon atoms, preferably between 2 and 5, carbon atoms, preferably containing at least one ethyl or butyl group and still more preferably $R_2O$ is a quaternary ammonium compound.

In a favoured embodiment the zeolite contains iron and/or chromium and its composition can be identified, terms of mole ratios of anhydrous oxides per 100 moles of silica as follows:

$$(0\text{—}10)M_{2/n}O:[(a)Cr_2O_3+(b)Fe_2O_3+(c)Al_2O_3]:100\ SiO_2,$$

wherein M is at least one cation having a valence n, a=0—4, b=0—5, c=0.001—0.5, with the proviso that a and b cannot both be equal to 0 at the same time; when one equals 0 the other must be greater than the value of c. The chromium and iron oxide need not occur as $Cr_2O_3$ or $Fe_2O_3$ but are so calculated in the formula.

In the as-synthesized form, this embodiment of the zeolite has the formula after dehydration, in terms of moles of oxides, per 100 moles of silica, as follows:

$$(0\text{—}3)R_2O:(0\text{—}8)M_{2/n}O:[(a)Cr_2O_3+(b)Fe_2O_3+(c)Al_2O_3]:100\ SiO_2,$$

wherein M is an alkali or alkaline earth metal, $R_2O$ is an organic compound of Group 5A element of the Periodic Table, preferably nitrogen or phosphorous, containing at least one alkyl or aryl group having between 1 and 7 carbon atoms, (preferably between 2 and 5, carbon atoms) preferably containing at least one butyl group and still more preferably $R_2O$ is a quaternary ammonium compound containing at least one butyl group, a=0—4, b=0—5, and c=0.001—0.4. However, a and b cannot both be equal to 0 at the same time. When one equals 0 the other must be greater than 0 and greater than the value of c.

The new form of the zeolite may have small amounts of Al, Fe and/or Cr in positions of tetrahedral substitution within the silica lattice. To this extent, the latter possesses a negative change, one excess electron for each atom substitution, which is balanced by cations. These cations may be replaced at least in part, by other ions using conventional ion exchange techniques. Due to pore blockage, in some cases, by the $R_2O$ species it may be necessary to pre-calcine the zeolite prior to ion-exchange. Ions introduced to replace the original alkali, alkaline earth and/or organic cations may be any that are desired so long as they can pass through the channels within the lattice. Especially desired replacing

ions are those of hydrogen, ammonium and metals of Groups I through VIII of the Periodic Table. Among the metals those particularly preferred are rare earth metals, manganese, zinc and those of Group VIII of the Periodic Table.

ZSM-11 has a characteristic X-ray diffraction pattern which distinguishes it from other crystalline materials and by which it is defined for the purposes of this specification.

The X-ray diffraction pattern of the zeolite has the following values:

TABLE 1

| Interplanar spacing D (A°) | Relative intensity |
|---|---|
| 11.2±.2 | m. |
| 10.1±.2 | m. |
| 6.73±.2 | w. |
| 5.75±.1 | w. |
| 5.61±.1 | w. |
| 5.03±.1 | w. |
| 4.62±.1 | w. |
| 4.39±.08 | w. |
| 3.86±.07 | vs. |
| 3.73±.07 | m. |
| 3.49±.07 | w. |
| (3.07, 3.00)±.05 | w. |
| 2.01±.02 | w. |

The parenthesis around lines 3.07 and 3.00 indicate that they are separate and distinct lines, but are often superimposed. These values were determined by standard techniques. The radiation was the K-alpha doublet of copper, and a Geiger counter spectrometer with a strip chart pen recorder was used. Peak heights and their positions as a function of 2 times theta, where theta is the Bragg angle, were read from the spectrometer chart. From these, the relative intensities, $100I/I_0$, where $I_0$ is the intensity of the strongest line or peak, and D the interplanar spacing in A°, corresponding to the recorded lines, were estimated. The intensity in the table above is expressed as follows:

m=medium, w=weak and vs=very strong

ZSM-11 is similar to ZSM-5 with the notable exception that whereas the ZSM-5 zeolite contains a doublet at about 10.1, 3.73, 3.00 and 2.01, A° interplaning spacing, ZSM-11 shows a singlet at these values. This means that the crystal class of the ZSM-11 is different from that of the other zeolite. ZSM-11 is tetragonal whereas ZSM-5 tends to be orthorhombic.

Different cationic forms reveal substantially the same pattern with minor shifts in interplanar spacing and variation of relative intensity.

Zeolite ZSM-11 can be used either in the alkali metal form, e.g. the sodium form, the ammonium form, the hydrogen form or another univalent or multivalent cationic form. When used as a catalyst it will usually first be subjected to thermal treatment to remove part or all of the organic constituent. It can also be used as a catalyst in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be exchanged into the composition to the extent A1 is in the structure, impregnated therein or physically intimately admixed therewith. Such component can be impregnated in or on to it by, in the case of platinum, treating the zeolite with a solution containing a platinum metal-containing ion. Thus, suitable platinum compounds include chloroplatinic acid, platinous chloride and various compounds containing the platinum amine complex.

The ammonium, alkylammonium and arylammonium forms can be converted to another form by thermal treatment, at a temperature of at least 700°F (371°C) for at least 1 minute and not greater than 20 hours. While subatmospheric pressure can be employed for the thermal treatment, atmospheric pressure is desired for reasons of convenience. The thermal treatment can be performed at a temperature up to about 1700°F (927°C). The thermally treated product is particularly useful in the catalysis of certain hydrocarbon conversion reactions.

When employed either as an absorbent or as a catalyst the zeolite should be dehydrated, at least partially. This can be done by heating to a temperature in the range of 200 to 600°C in an atmosphere, such as air, nitrogen, etc. and at atmospheric, subatmospheric or superatmospheric pressures for between 1 and 48 hours. Dehydration can also be performed at room temperature merely by placing the ZSM-11 in a vacuum, but a longer time is required to obtain a sufficient amount of dehydration.

ZSM-11 according to the present invention can be prepared from a reaction mixture containing a source of silica, $R_2O$, an alkali metal oxide, e.g. sodium, water, and no added alumina, and having a composition, in terms of mole ratios of oxides, falling within the following ranges:

| Reactants | Broad | Preferred |
|---|---|---|
| $SiO_2/R_2O$ | =5 to 30 | 10 to 20 |
| $M_2O/R_2O$ | =0.0 to 6 | 0.09 to 3.0 |
| $H_2O/R_2O$ | =100 to 500 | 300 to 400 |

wherein $R_2O$ is the oxide form of an organic compound of an element of Group 5-A of the Periodic Table and can be a compound containing one butyl group, M is an alkali or alkaline earth metal, and maintaining the mixture, at crystallization temperatures, until crystals are formed. No alumina is added, the only aluminium present occurring as an impurity in some other component of the crystallization medium.

The chromium and/or iron containing form of the zeolite can be prepared from a reaction mixture containing a source of silica, $R_2O$, an alkali metal oxide, e.g. sodium, a chromium or iron compound, water, and no added alumina, and having a composition, in terms of mole ratios of oxides, falling within the following ratios:

| Reactants | Broad | Preferred |
|---|---|---|
| $SiO_2/R_2O$ | =5 to 30 | 10 to 20 |
| $M_2O/R_2O$ | =0 to 6 | 0.9 to 3.0 |
| $(Cr_2O_3+Fe_2O_3)/R_2O$ | =0.2 to 1.0 | 0.2 to 0.4 |
| $H_2O/R_2O$ | =100 to 500 | 300 to 400 |

wherein $R_2O$ is the oxide form of an organic compound of an element of Group 5-A of the Periodic Table and can be a compound containing one butyl group, and M is an alkali or alkaline earth metal, and maintaining the mixture until crystals are formed. Again, no alumina is added.

Preferably, crystallization is performed under pressure in an autoclave or static bomb reactor but can be carried out in polypropylene jars at 100°C. The temperature may range from 80°C to 250°C, preferably 100°C to 200°C, but at lower temperatures e.g. about 100°c, crystallization time is longer. These times vary from about 6 hrs to 90 days. Thereafter, the crystals are separated from the liquid and recovered. The composition can be prepared utilizing materials which supply the appropriate oxide. Such compositions include sodium silicate, silica hydrosol, silica gel, silicic acid, sodium hydroxide, chromic potassium sulphate and ferric ammonium sulphate. The organic compounds can be any element of Group 5-A such as nitrogen, phosphorus, arsenic, antimony. The preferred compounds are quaternary compounds generally expressed by the following formula:

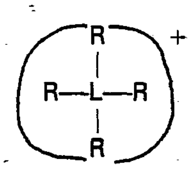 or $R_4L$

wherein L is an element of Group 5-A of the Periodic Table, and each R is an alkyl or aryl group having between 1 and 7 (preferably 2—5) carbon atoms. Preferably at least one R group is butyl. While normally each alkyl or aryl group will be the same, it is not necessary that each group have the same number of carbon atoms in the chain. In preparing an ammonium species, the organic substituted ammonium chloride or less preferably, hydroxide is useful. In preparing the phosphonium species of the zeolite tetrabutylphosphonium chloride is particularly desirable as a means of incorporating the quaternary metal compound in the zeolite. In preparing an ammonium species tetrabutylammonium chloride or bromide or, less preferably, hydroxide is useful. The other elements of Group 5-A behave similarly and thus the zeolite containing the same can be prepared by the same manipulative procedure substituting the other Group 5-A metal for phosphorous. It should be realized that the oxide can be supplied from more than one source. The reaction mixture can be prepared either batchwise or continuously. Crystal size and crystallization time will vary with the nature of the reaction mixture employed and the crystallization conditions.

The quaternary compounds need not be used as such. They may be produced *in situ* by the addition of the appropriate precursors. These precursors comprise a compound characterized by the formula $R_1R_2R_3L$ where $R_1$, $R_2$ and $R_3$ are selected from alkyl, substituted alkyl, aryl substituting aryl, cycloalkyl, substituted cycloalkyl and hydrogen and L is an element of Group 5-A and a compound of the formula $R_4$ where $R_4$ is alkyl, substituted alkyl, cycloalkyl, substituting cycloalkyl, aryl and substituted aryl and X is an electronegative group. According to a special embodiment of the invention, the method of the invention can be practised using the compound $R_1R_2R_3L$ alone. In this case it is assumed that quaternary compounds are not formed. Thus, in specific embodiments one may use as the source of $R_2O$, amines or phosphines either primary, secondary or tertiary as well as diamines without addition of any $R_4X$.

Synthesis can be facilitated by the presence of at least 0.001, preferably at least 0.01, more preferably at least 0.1 percent by weight (based on total crystalline product) of seed crystals. The zeolite may be formed in a wide variety of particular sizes. Generally speaking, the particles can be in the form of a powder, a granule, or a molded product, such as an extrudate having particle size sufficient to pass through a 2 mesh (Tyler) screen and be retained on a 400 mesh (Tyler) screen. In cases where a catalyst is molded, such as by extrusion, the zeolite can be extruded before drying or dried partially dried and then extruded.

When used as a catalyst ZSM-11 according to the invention may be composited with a matrix, as described in relation to zeolite ZSM-43 in our European specification 0,001,695.

Employing the zeolite of this invention as a catalyst, which may contain additional hydrogenation components, heavy petroleum residual stocks, cycle stocks, and other hydrocrackable charge stocks can be hydrocracked at temperatures between 204°C (400°F) and 435°C (815°F) using molar ratios of hydrogen to hydrocarbon charge in the range between 2 and 80. The pressure employed will vary between 170 and 17338 kPa (10 and 2,500 psig) and the liquid hourly space velocity between 0.1 and 10.

Hydrocarbon cracking stocks can be cracked at a liquid hourly space velocity between about 0.5 and 50, a temperature between about 287°C (550°F) and 593°C (1100°F), a pressure between about subatmospheric and several hundred atmospheres.

Reforming stocks can be reformed employing a temperature between 371°C (700°F) and 537°C (1000°F). The pressure can be between 790 and 6996 kPa (100 and 1000 psig) but is preferably between 1480 and 4927 kPa (200 and 700 psig). The liquid hourly space velocity is generally between 0.1 and 10, preferably between 0.4 and 4 and the hydrogen to hydrocarbon mole ratio is generally between 1 and 20 preferably between 4 and 12.

The zeolite can also be used for hydroisomerization of normal paraffins, when provided with a hydrogenation component, e.g., platinum. Hydroisomerization is carried out at a temperature between 93° and 371°C (200° and 700°F), preferably 148 to 287°C (300° to 550°F), with a liquid hourly space velocity 0.01 and 2, preferably between 0.25 and 0.50 employing hydrogen such that the hydrogen to hydrocarbon mole ratio is between 1:1 and 5:1. Additionally, the catalyst can be used for olefin isomerization employing temperatures between −1°C and 371°C (30°F and 700°F).

Other reactions which can be accomplished employing the catalyst of this invention containing a metal, e.g., platinum, include hydrogenation-dehydrogenation reactions and desulfurization reactions, olefin polymerization (oligomerization) and other organic compound conversions such as the conversion of alcohols (e.g. methanol to hydrocarbon).

In the Examples which follow adsorption data were determined as follows:

A weighed sample of the calcined zeolite was contacted with the desired pure adsorbate vapor in an adsorption chamber, evacuated to <1 mm and contacted with 12 mm Hg of water vapor and 20 mm Hg of cyclohexane and n-hexane vapor, pressures less than the vapor-liquid equilibrium pressure of the respective adsorbate at room temperature. The pressure was kept constant (within about ±0.5 mm) by addition of adsorbate vapor controlled by a manostat during the adsorption period, which did not exceed about eight hours. As the adsorbate was adsorbed the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant.

Examples 1—4

A starting gel reaction mixture was prepared from sodium silicate (28.8% $SiO_2$, 8,9% $Na_2O$, 62% $H_2O$), obtained commercially as "Q-brand", tetrabutylammonium bromide, sulfuric acid and water. Crystallization was carried out in polypropylene jars at 98°C (210°F) or in 2 litre stainless steel pressure vessels (121°C (250°F)). After crystallization, the solids were separated from any unreacted components by filtration and then water washed followed by drying at 110°C (230°F). The amounts of starting materials, identification of same, product compositions, and adsorption data are listed in Table 2.

Examples 5—15

A starting gel mixture was prepared from colloidal silica, tetrabutylammonium bromide, sodium hydroxide and water. Crystallization was carried out in propylene jars at 98°C (210°F) or in 2 litre stainless steel pressure vessels (148°C (300°F)). After crystallization, the solids were separated from any unreacted components by filtration and then water washed followed by drying at 110°C (230°F). The amounts of starting materials, identification of same, product compositions, adsorption data, and catalytic data are listed in Tables 3 (Examples 5—8) and 3A (Examples 9—15 and 18).

Example 16

A starting gel mixture was prepared from fume silica (91.3% $SiO_2$), tetrabutylammonium bromide, sodium hydroxide and water. Crystallization was carried out in propylene jars at 98°C (210°F). After

crystallization, the solids were separated from any unreacted components by filtration and then water washed followed by drying at 110°C (230°F). The amounts of starting materials, identification of same, product composition, and catalytic data are listed in Table 4.

Example 17

A starting gel mixture was prepared from colloidal silica, tetrabutylammonium bromide and hydroxide (40%) and water. Crystallization was carried out in propylene jars at 98°C (210°F). After crystallization the solids were separated from any unreacted components by filtration and then water washed followed by drying at 110°C (230°F). The amounts of starting materials, identification of same, product composition, and catalytic data are listed in Table 5.

The data in Tables 2—5 show that the $Al_2O_3$ content (after drying) of the as-synthesized materials varied from 0.04 (Example 18) to 0.64% (Example 16). Most, however, ranged from 0.05 to 0.2 (Examples 1—3, 5—10, 12 and 14—17). It was also found that synthesis in the presence of ZSM-11 seeds yielded products of high crystallinity, as high as 130% in three days (Example 14).

Examples 19—22

These examples illustrate the use of tetrabutylphosphonium chloride as the source of the alkyl Group 5-A cation, and one example (22) additionally incorporates triethanol amine (to promote larger crystal growth) into the starting mixture, which is otherwise the same as that of Examples 1—4. The amounts of starting materials, identification of same, product composition, adsorption data, and catalytic data are listed in Table 6.

The data presented in Table 6 show that the $Al_2O_3$ content (after drying) of the as-synthesized materials was negligible. It is to be noted, with respect to Example 22, that not only was triethanol amine introduced to promote larger crystal growth, but crystallization was allowed to proceed for 50 days. X-ray analysis was about 100% ZSM-11 type zeolite.

Examples 23—34

A starting gel reaction mixture was prepared from sodium silicate (28.8% $SiO_2$, 8.9% $Na_2O$, 62% $H_2O$), tetrabutylammonium bromide, chromic potassium sulfate, sulfuric acid and water. Crystallization was carried out in polypropylene jars at 98°C (210°F), glass-lined stainless steel autoclaves (148°C (300°F)) or in stirred 2 liter stainless steel pressure vessels at 121—148°C (250—300°F). After crystallization the solids were separated by filtration, water washed, and dried at 110°C (230°F). The amounts of starting material, identification of same, product compositions and adsorption data are listed in Table 7.

Examples 35—36

A starting gel mixture was prepared from sodium silicate (28.0% $SiO_2$, 8.9% $Na_2O$, 62% $H_2O$) tetrabutylammonium bromide, ferric ammonium sulfate, sulfuric acid and water. Crystallization was carried out in polypropylene jars at 98°C (210°F) or in stirred 2 liter stainless steel pressure vessels (148°C (300°F)). After crystallization the solids were filtered, water washed, and dried at 110°C (230°F). The amounts of starting materials, identification of same, product compositions and adsorption data are listed in Table 8.

Example 37

This example illustrates its preparation of a low alumina ZSM-11 through the use of a specially prepared high purity silica hydrogel. The silica hydrogel used as a source of silica was prepared as follows:

A. 2479 cc of tetraethylorthosilicate $Si(OCH_2CH_3)4$
782 cc ethanol
178.7 cc 4N $HNO_3$

Allowed to age for 1/2 hour at room temperature, then cooled to 3—6°C (38—43°F).

B. 233.3 cc $NH_4OH$, cooled to 3—6°C (38—43°F).

Solution B is added to A and mixed for 30 seconds. The resultant gel is water washed and has 17.0% solids.

Preparational details are given in Table 9.

The data in Tables 7, 8 and 9 show that the $Al_2O_3$ content (after drying) of the as-synthesized materials varied from 370 ppm (pars per million) (Example 37) to 0.30% (Example 31). Most, however ranged between 0.2 to 0.3 (Examples 23—30, 32, 33 and 34). It was also found that synthesis in the presence of ZSM-11 seed yielded products of high crystallinity, as high as 115% in five days (Example 36).

Examples 38—44

Aliquots of the products of some of the previous Examples were calcined at 537°C (1000°F) and then ion exchanged with a solution of $NH_4Cl$, followed by recalcination of the $NH_4$ ZSM-11 to the H-form, of which the sodium content was:

| Example | Base material (material of) | Na content, (wt %) |
|---|---|---|
| 38 | Example 7 | 0.02 |
| 39 | Example 15 | 0.02 |
| 40 | Example 9 | <0.01 |
| 41 | Example 6 | <0.01 |
| 42 | Example 3 | <0.01 |
| 43 | Example 14 | 0.02 |
| 44 | Example 12 | .034 |

A 1.0 cc sample of each of the aliquots was tested for cracking of n-hexane at a liquid hourly space velocity of 1.0 and a temperature of 537°C (1000°F). The % n-hexane conversion and the calculated relative cracking activity $\alpha$ of the catalysts after 5 minutes on stream were:

| Example | Conversion | $\alpha$ |
|---|---|---|
| 38 | 2.3 | 0.4 |
| 39 | 3.1 | 0.6 |
| 40 | 1.6 | 0.3 |
| 41 | 3.6 | 0.7 |
| 42 | 10.3 | 2.1 |
| 43 | 2.9 | 0.6 |
| 44 | 0.9 | 0.3 |

The $\alpha$-test is described in a letter to the editor entitled "Superactive Crystalline Aluminosilicate Hydrocarbon Cracking Catalysts" by P. B. Weisz and J. N. Miale, Journal of Catalysts, Vol. 4, pp. 527—529 (August 1965).

Most of the aliquots were also tested for oligomerization of propylene. In this test propylene was passed over either a 0.25 g or a 0.5 g sample of the catalyst as a design flow ratio of 1000 cc/hr and a nominal temperature of 371°C (700°F). The resulting weight hourly space velocity was about 3.5 for the 0.50 g loading and 7 for the 0.25 g loading. The actual WHSV and catalytic data calculated on a total recovery basis were:

| Example | WHSV | Conversion wt % | Selectivity % on $C_3$ converted $C_4$+ | $C_5$+ | $C_6$+ |
|---|---|---|---|---|---|
| 38 | 3.8 | 93.2 | 94.8 | 68.5 | 48.1 |
| 39 | 3.7 | 91.5 | 95.5 | 70.3 | 47.3 |
| 40 | 3.7 | 90.4 | 96.0 | 70.8 | 47.8 |
| 41 | 3.7 | 92.8 | 93.6 | 69.7 | 50.8 |
| 42 | 7.5 | 91.8 | 93.4 | 64.9 | 43.8 |
| 43 | 7.4 | 85.4 | 96.9 | 62.9 | 36.8 |

Examples 45—50

Aliquots of the products of Examples 23, 26, 27, 31 and 36 were subjected to the $\alpha$ test for cracking activity. In addition, the products of Examples 23 and 26 were tested for propylene oligomerization. Both tests were those employed in Examples 38 to 44, all pertinent details, i.e. conditions of treatment of the catalyst, compositions after treatment and calcination, test results and the like, are set forth in Table 10.

The data set forth in Table 10 reveals that n-Hexane conversion after 5 minutes ranged from 0.3 (Example 48) to 17.1 (Example 46) wt per cent. $\alpha$ values ranged after 5 minutes from 0.06 (Example 48) to 3.6 (Example 46). The best n-hexane conversion and $\alpha$-values were obtained from the product of original Example 26.

TABLE 2

| Example | 1 | 2 | 3 | 4 |
|---|---|---|---|---|
| Starting materials, gms | | | | |
| A. Sodium silicate (28.8%, $SiO_2$, 8.9% $Na_2O$, 62% $H_2O$) | 70.0 | 140 | 455 | 70.0 |
| $H_2O$ Colloidal $SiO_2$ (30% $SiO_2$) NaOH | 80.0 | 160 | 520 | 80.0 |
| B. Tetrabutylammonium bromide | 17.4 | 34.8 | 113.1 | 17.4 |
| $H_2SO_4$ | 7.2 | 14.4 | 46.3 | 3.6 |
| $H_2O$ | 80.0 | 160 | 520 | 80.0 |
| C. ZSM-11 Seeds (82.9%) | | | | |
| Starting Compositions, moles | | | | |
| $(TBA)_2O$ (1) | 1.0 | 1.0 | 1.0 | 1.0 |
| $Na_2O$ | 1.0 | 1.0 | 1.0 | 2.28 |
| $Na_2SO_4$ | 2.57 | 2.57 | 2.57 | 1.28 |
| $SiO_2$ | 12.0 | 12.0 | 12.0 | 12.0 |
| $H_2O$ | 403 | 403 | 403 | 403 |
| Crystallization Conditions | | | Stirred | |
| Temp °C (°F) | 98 (210) | 98 (210) | 121 (250) | 98 (210) |
| Time, Days | 15 | 15 | 5 | 16 |
| X-Ray Analysis | ZSM-11 105% | ZSM-11 105% | ZSM-11 150% | ZSM-11 110% |
| Product Compositions wt. % | | | | |
| C | | | 8.97 | |
| N | 0.89 | 0.66 | 0.65 | 0.67 |
| Na | 1.36 | 1.30 | 1.40 | 2.15 |
| $SiO_2$ | 81.04 | 80.07 | 82.1 | 79.8 |
| Ash | 85.9 | 83.4 | 84.9 | 84.5 |
| $Al_2O_3$ | 0.18 | 0.21 | 0.16 | 0.33 |
| Molar Ratio $SiO_2/Al_2O_3$ | 765 | 648 | 872 | 411 |
| Adsorption wt. % (537°C 1000°F) Calcination) | | | | |
| Cyclohexane | 7.1 | 6.2 | 3.8 | 3.7 |
| n-Hexane | 10.0 | 9.5 | 8.2 | 8.4 |
| $H_2O$ | 13.5 | 14.4 | 8.4 | 11.8 |
| Surface Area $m^2/gm$ | 327 | 328 | 256 | |

(1) TBA=Tetrabutylammonium$^+$.

TABLE 3

| Example | 5 | 6 | 7 | 8 |
|---|---|---|---|---|
| Starting Materials, gms | | | | |
| A. Sodium Silicate (28.8% $SiO_2$, 8.9% $Na_2O$, 62% $H_2O$) $H_2O$ | 50.0 | 100 | 50.0 | 280 |
| Colloidal $SiO_2O$ (30% $SiO_2$) | 190 | 380 | 190 | 1064 |
| NaOH | 14.6 | 29.2 | 14.6 | 81.76 |
| B. Tetrabutylammonium Bromide | 19.0 | 38.0 | 19.0 | 106.4 |
| $H_2SO_4$ | | | | |
| $H_2O$ | 24.0 | 48.0 | 24.0 | 134 |
| C. ZSM-11 Seeds (82.9%) | 0.6 | 0.5 | | 1.68 |
| Starting Compositions, moles | | | | |
| $(TBA)_2O$ (1) | 1.0 | 1.0 | 1.0 | 1.0 |
| $Na_2O$ | 6.2 | 6.2 | 6.2 | 6.2 |
| $Na_2SO_4$ | | | | |
| $SiO_2$ | 32.2 | 32.2 | 32.2 | 32.2 |
| $H_2O$ | 390 | 390 | 390 | 390 |
| Crystallization Conditions | | | | |
| Temp °C (°F) | 98 (210) | 98 (210) | 98 (210) | 121 (250) |
| Time, Days | 11 | 12 | 18 | 7 |
| X-Ray Analysis | ZSM-11 135% | ZSM-11 135% | ZSM-11 120% | ZSM-11 145% |
| Product Compositions wt. % | | | | |
| C | | | | 7.90 |
| N | 0.66 | 0.85 | 1.16 | 0.57 |
| Na | 1.33 | 1.75 | 1.70 | 2.0 |
| $SiO_2$ | 78.7 | 82.0 | 80.45 | 83.2 |
| Ash | 85.5 | 85.3 | 85.2 | 85.9 |
| $Al_2O_3$ | 0.10 | 0.10 | 0.09 | 0.12 |
| Molar Ratio $SiO_2/Al_2O_3$ | 1338 | 1394 | 1520 | 1176 |
| Adsorption wt. % (537°C 1000°F) Calcination) | | | | |
| Cyclohexane | 5.9 | | 7.0 | 3.4 |
| n-Hexane | 10.0 | | 10.6 | 7.3 |
| $H_2O$ | 13.3 | | 13.2 | 9.4 |
| Surface Area $m^2/gm$ | 336 | | 367 | 227 |

(1) TBA=Tetrabutylammonium$^+$.

TABLE 3A

| Examples | 9 | 10 | 11 | 12 | 13 | 14 | 15 | 18 |
|---|---|---|---|---|---|---|---|---|
| Starting Materials, gms | | | | | | | | |
| A. $H_2O$ | 50.0 | 285 | 90.0 | 540 | 90.0 | 540 | 160 | 50 |
| Colloidal $SiO_2$ (10% $SiO_2$) | 190 | 1080 | 76.0 | 456 | 76.0 | 456 | 152 | 380 |
| NaOH | 7.3 | 41.6 | 2.4 | 14.4 | 2.4 | 14.4 | 4.8 | 7.3 |
| Fume Silicon (91.3%) | | | | | | | | |
| B. Tetrabutylammonium Bromide | 19.0 | 108.3 | 18.0 | 108 | 18.0 | 108 | 36.0 | 19.0 |
| $H_2O$ | 24.0 | 137 | 90.0 | 540 | 90.0 | 540 | 160 | 24.0 |
| C. ZSM-11 Seeds (82.9%) | | | | | | | | |
| Starting Composition, moles | | | | | | | | |
| $(TBA)_2O$ (1) | 1.0 | 1.0 | 1.0 | 1.0 | 1.0 | 1.0 | 1.0 | 1.0 |
| $Na_2O$ | 3.1 | 3.1 | 1.07 | 1.07 | 1.07 | 1.07 | 1.07 | 3.1 |
| $SiO_2$ | 32.2 | 32.2 | 13.6 | 13.6 | 13.6 | 13.6 | 13.6 | 64.4 |
| $H_2O$ | 390 | 390 | 462 | 462 | 462 | 462 | 423 | 640 |
| Crystallization Conditions | | Stirred | | Stirred | | Stirred | | |
| Temp°C (°F) | 98 (210) | 121 (250) | 98 (210) | 121 (250) | 98 (210) | 121 (250) | 98 (210) | 98 (210) |
| Time, Days | 21 | 7 | 19 | 7 | 5 | 3 | 7 | 24 |
| X-Ray Analysis | ZSM-11 110% | ZSM-11 125% | ZSM-11 105% | ZSM-11 120% | ZSM-11 90% | ZSM-11 130% | ZSM-11 100% | ZSM-11 110% |
| Product Composition, wt. % | | | | | | | | |
| C | | 9.60 | | 10.0 | | 1.0 | | |
| N | 1.02 | 0.67 | | 0.72 | | 0.71 | 1.41 | 0.65 |
| Na | 1.42 | 2.70 | | 1.34 | | 1.15 | 1.09 | 0.67 |
| $SiO_2$ | 79.23 | 78.71 | | 83.57 | | 80.67 | 79.6 | 82.9 |
| Ash | 84.9 | 83.4 | | 83.59 | | 84.41 | 84.2 | 86.8 |
| $Al_2O_3$ | 0.05 | 0.08 | | 0.05 | | 0.09 | 0.07 | 0.04 |
| Molar Ratio $SiO_2/Al_2O_3$ | 2694 | 1673 | | 2841 | | 1524 | 1933 | 3520 |
| Adsorption wt. % (537°C 1000°F) Calcination) | | | | | | | | |
| Cyclohexane | 5.9 | 1.4 | | 4.9 | | 4.1 | 7.4 | 9.0 |
| n-Hexane | 9.5 | 5.5 | | 10.0 | | 10.2 | 10.1 | 11.4 |
| $H_2O$ | 13.8 | 16.1 | | 10.5 | | 10.3 | 15.0 | 13.4 |
| Surface Area, $m^2/gm$ | 298 | 137 | | 298 | | | 332 | |

(1) TBA=Tetrabutylammonium.

TABLE 4

| Example | 16 |
|---|---|
| Starting Materials, gms | |
| A. $H_2O$ | 195 |
| Colloidal $SiO_2$ (30% $SiO_2$ | |
| NaOH | 5.04 |
| Fume Silica (91.3%) | 48.0 |
| B. Tetrabutylammonium Bromide | 13.1 |
| $H_2O$ | 48.0 |
| C. ZSM-11 Seeds (82.9%) | |
| Starting Composition, moles | |
| $(TBA)_2O$ (1) | 1.0 |
| $Na_2O$ | 3.1 |
| $SiO_2$ | 36.0 |
| $H_2O$ | 665 |
| Crystallization Conditions | |
| Temp °C (°F) | 98 (210) |
| Time, Days | 41 |
| X-Ray Analysis | ZSM-11 100% |
| Product Composition, wt. % | |
| C | |
| N | 0.63 |
| Na | 1.29 |
| $SiO_2$ | 77.09 |
| Ash | 83.13 |
| $Al_2O_3$ | 0.64 |
| Molar Ratio $SiO_2/Al_2O_3$ | 205 |
| Adsorption, wt. % (537°C 1000°F) Calcination) | |
| Cyclohexane | 6.4 |
| n-Hexane | 8.1 |
| $H_2O$ | 4.2 |
| Surface Area, $m^2/gm$ | 312 |

(1) TBA=Tetrabutylammonium

TABLE 5

| Example | 17 |
|---|---|
| Starting Materials, gms | |
| A. $H_2O$ | |
| Colloidal $SiO_2$ (30% $SiO_2$) | 132.8 |
| NaOH | |
| Fume Silica (91.3%) | 40% TBAOH (2) |
| B. Tetrabutylammonium Bromide | 64.8 |
| $H_2O$ | 34.2 |
| C. ZSM-11 Seeds (82.9%) | 0.3 |
| Starting Compositions, moles | |
| $(TBA)_2O$ (1) | 1.0 |
| $Na_2O$ | 0.0 |
| $SiO_2$ | 13.3 |
| $H_2O$ | 180 |

TABLE 5 (contd.)

| | |
|---|---|
| Crystallization Conditions | |
| Temp (°F) °C | (210) 98 |
| Time, Days | 9 |
| X-Ray Analysis | ZSM-11 |
| | 80% |
| Product Composition, wt. % | |
| C | |
| N | 1.27 |
| Na | 0.16 |
| $SiO_2$ | 78.1 |
| Ash | 80.7 |
| $Al_2O_3$ | 0.09 |
| Molar Ratio $SiO_2/Al_2O_3$ | 1475 |
| Adsorption, wt. % (537°C 1000°F) Calcination) | |
| Cyclohexane | 13.8 |
| n-Hexane | 14.3 |
| $H_2O$ | 29.1 |
| Surface Area, $m^2/gm$ | 522 |

(1) TBA=Tetrabutylammonium
(2) TBAOH=Tetrabutylammonium hydroxide solution (40%).

TABLE 6

| Examples | 19 | 20 | 21 | 22 |
|---|---|---|---|---|
| Starting Materials, gms | | | | |
| A. Sodium Silicate (28.8% $SiO_2$, 8.9% $Na_2O$, 62% $H_2O$) | 50 | 150 | 300 | 50 |
| $H_2O$ | 250 | 750 | 1500 | 600 |
| B. Tetrabutylphosphonium chloride | 25 | 75 | 150 | 25 |
| $H_2SO_4$ (96%) | 5 | 15 | 30 | 4.8 |
| C. Triethanol Amine | | | | 100 |
| Starting Composition, moles | | | | |
| $(TBP)_2O$ (1) | .0424 | .1272 | .2544 | .0424 |
| $Na_2O$ | .0718 | .2154 | .4308 | .0718 |
| $SiO_2$ | .2396 | .7188 | 1.4376 | .2396 |
| $H_2O$ | 15.61 | 46.83 | 93.66 | 35.06 |
| X-Ray Analysis | ZSM-11 | ZSM-11 | ZSM-11 | ZSM-11 |
| | 95% | 90% | 105% | 100% |
| Crystallization Conditions | | | | |
| Temp °C (°F) | 100 (212) | 100 (212) | 100 (212) | 100 (212) |
| Time, Days | 19 | 10 | 11 | 50 |
| Product Composition, wt. % | | | | |
| C | | | | |
| P | .54 | .50 | .44 | .22 |
| Na | 1.1 | 1.79 | | .39 |
| $SiO_2$ | 96.4 | 99.5 | 94.3 | 93.7 |
| $Al_2O_3$ | .14 | .15 | .13 | .17 |
| Molar Ratio $SiO_2/Al_2O_3$ | 1169 | 1069 | 1257 | 949 |

(1) TBP=Tetrabutylphosphonium$^+$.

TABLE 7

| Examples | 23 | 24 | 25 | 26 | 27 | 28 |
|---|---|---|---|---|---|---|
| **Starting Materials-gms** | | | | | | |
| A. Na Silicate (28.8% $SiO_2$, 8.9% $Na_2O$, 62.0% $H_2O$ | 74.6 | 74.6 | 74.6 | 37.3 | 74.6 | 74.6 |
| $H_2O$ | 52.5 | 60.0 | 52.5 | 30.0 | 52.5 | 52.5 |
| B. $CrK(SO_4)_2$ $12H_2O$ | 9.2 | 9.2 | 9.2 | 4.6 | 9.2 | 9.2 |
| $H_2SO_4$ | 2.1 | 4.2 | 2.1 | 1.0 | 4.2 | 2.1 |
| $H_2O$ | 105 | 105 | 105 | 53.0 | 105 | 105 |
| C. Tetrabutylammonium bromide | 20.5 | 20.0 | 20.5 | 10.2 | 20.5 | 20.5 |
| D. ZSM-11 Seeds (gms. solids) | | | | | | |
| **Starting Composition moles** | | | | | | |
| $(TBA)_2O$ (1) | 1.0 | 1.0 | 1.0 | 1.0 | 1.0 | 1.0 |
| $Na_2O$ | 1.54 | 0.91 | 1.54 | 1.58 | 0.89 | 1.54 |
| $Na_2SO_4$ | 1.82 | 2.54 | 1.82 | 1.80 | 2.48 | 1.82 |
| $Cr_2O_3$ | 0.289 | 0.30 | 0.289 | 0.29 | 0.289 | 0.289 |
| $K_2O$ | 0.289 | 0.30 | 0.289 | 0.29 | 0.289 | 0.289 |
| $SiO_2$ | 11.26 | 11.55 | 11.26 | 11.33 | 11.26 | 11.26 |
| $H_2O$ | 355 | 378 | 353 | 373 | 355 | 355 |
| **Crystallization Conditions** | | | | | | |
| Temp °C (°F) | 98 (210) | 98 (210) | 98 (210) | 98 (300) | 98 (210) | 98 (210) |
| Days | 60 | 48 | 84 | 17 | 66 | 44 |
| X-ray Analysis (3) | ZSM-11 70% | ZSM-11 70% | ZSM-11 75% | ZSM-11 135% trace ZSM-5 | ZSM-11 75% | ZSM-11 85% |
| **Product Composition, wt. %** | | 537°C | (1000°F) | | | |
| C | | | | | | |
| N | | | — | | | 0.52 |
| Na | | | 1.34 | | | 1.66 |
| Cr | | | 0.83 | | | 2.50 |
| K | | | 0.39 | | | 2.20 |
| $SiO_2$ | | | 85.2 | | | 76.3 |
| Ash | | | 96.6 | | | 85.2 |
| $Al_2O_3$ | | | 0.23 | | | 0.178 |

TABLE 7 (contd.)

| Examples | 23 | 24 | 25 | 26 | 27 | 28 |
|---|---|---|---|---|---|---|
| Product Composition, moles (5) | | | | | | |
| $R_2O$ | | | — | | | 1.46 |
| $Na_2O$ | | | 2.05 | | | 2.84 |
| $K_2O$ | | | 0.35 | | | 2.21 |
| $Cr_2O_3$ | | | 0.56 | | | 1.89 |
| $Al_2O_3$ | | | 0.16 | | | 0.13 |
| $SiO_2$ | | | 100 | | | 100 |
| Molar Ratio $SiO_2/Al_2O_3$ | | | 625 | | | 769 |
| Adsorption, wt. % | | | | | | 1000°F |
| Cy-$C_6$ | | | | | | 3.4 |
| n-$C_6$ | | | | | | 7.0 |
| $H_2O$ | | | | | | 32.8 |
| Surface Area, $m^2/gm$ | | | | | | 205 |

TABLE 7 (contd.)

| Examples | 29 | 30 | 31 | 32 | 33 | 34 |
|---|---|---|---|---|---|---|
| Starting Materials-gms | | | | | | |
| A. Na Silicate (28.8% $SiO_2$, 8.9% $Na_2O$, 62.0% $H_2O$ | 484.9 | 484.9 | 484.9 | 484.9 | 484.9 | 484.9 |
| $H_2O$ | 390.0 | 390.0 | 390.0 | 390.0 | 367.5 | 390 |
| B. $CrK(SO_4)_2$ $12H_2O$ | 59.8 | 59.8 | 59.8 | 59.8 | 64.4 | 59.8 |
| $H_2SO_4$ | 13.0 | 13.0 | 13.0 | 13.0 | 29.4 | 13.0 |
| $H_2O$ | 689 | 689 | 689 | 689 | 735 | 689 |
| C. Tetrabutylammonium bromide | 132.6 | 132.6 | 132.6 | 132.6 | 143.5 | 132.6 |
| D. ZSM-11 Seeds (gms. solids) | | | 2.0 | 2.0 | 2.2 | |
| Starting Composition moles | | | | | | |
| $(TBA)_2O$ (1) | 1.0 | 1.0 | 1.0 | 1.0 | 1.0 | 1.0 |
| $Na_2O$ | 1.58 | 1.58 | 1.58 | 1.58 | 0.90 | 1.58 |
| $Na_2SO_4$ | 1.80 | 1.80 | 1.80 | 1.80 | 2.48 | 1.80 |
| $Cr_2O_3$ | 0.291 | 0.291 | 0.291 | 0.291 | 0.289 | 0.291 |
| $K_2O$ | 0.291 | 0.291 | 0.291 | 0.291 | 0.291 | 0.291 |
| $SiO_2$ | 11.33 | 11.33 | 11.33 | 11.33 | 11.23 | 11.33 |
| $H_2O$ | 373 | 373 | 373 | 373 | 356 | 373 |

TABLE 7 (contd.)

| Examples | 29 | 30 | 31 | 32 | 33 | 34 |
|---|---|---|---|---|---|---|
| Crystallization Conditions | Stirred (4) | | Stirred (4) | | Stirred (4) | Stirred (4) |
| Temp °C (°F) | 148 (300) | 104 (220) | 148 (300) | 121 (250) | 121 (250) | 121 (250) |
| Days | 5 | 10 | 5 | 10 | 7 | 5 |
| X-ray Analysis (3) | ZSM-11 90% | ZSM-11 86% | ZSM-11 100% | ZSM-11 85% | ZSM-11 90% | ZSM-11 95% |
| Product Composition, wt. % | | | | | | |
| C | 7.08 | 6.75 | 7.32 | 7.24 | 7.77 | 7.25 |
| N | 0.37 | 0.51 | 0.59 | 0.51 | 0.54 | 0.51 |
| Na | 2.4 | 1.30 | 2.31 | 2.49 | 2.45 | 1.85 |
| Cr | 5.6 | 4.7 | 2.7 | 3.8 | 2.9 | 4.0 |
| K | 0.62 | 0.48 | 0.47 | 0.52 | 0.47 | 0.37 |
| $SiO_2$ | 82.8 | 84.4 | 82.6 | 83.0 | 74.4 | 71.58 |
| Ash | 96.9 | 97.6 | 98.6 | 98.1 | 87.1 | 86.6 |
| $Al_2O_3$ | 0.26 | 0.25 | 0.30 | 0.29 | 0.26 | 0.27 |
| Production Composition, moles (5) | | | | | | |
| $R_2O$ | 0.95 | 1.29 | 1.54 | 1.31 | 1.55 | 1.53 |
| $Na_2O$ | 3.78 | 2.01 | 3.65 | 3.91 | 4.29 | 3.38 |
| $K_2O$ | 0.58 | 0.44 | .44 | 0.48 | 0.48 | 0.40 |
| $Cr_2O_3$ | 3.90 | 3.21 | 1.88 | 2.64 | 2.25 | 3.22 |
| $Al_2O_3$ | 0.18 | 0.17 | 0.21 | 0.21 | 0.20 | 0.23 |
| $SiO_2$ | 100 | 100 | 100 | 100 | 100 | 100 |
| Molar Ratio $SiO_2/Al_2O_3$ | 555 | 588 | 476 | 476 | 500 | 434 |
| Adsorption, wt. % | (1000°F) 537°C | (1000°F) 537°C | (1000°F) 537°C | (1000°F) 537°C | (1000°F) 537°C | (1000°F) 537°C |
| Cy-$C_6$ | 1.4 | 1.9 | 1.5 | 2.3 | 2.6 | 1.1 |
| n-$C_6$ | 1.9 | 6.0 | 4.4 | 5.6 | 5.7 | 3.5 |
| $H_2O$ | 9.1 | 31.5 | 28.7 | 26.3 | 31.1 | 29.5 |
| Surface Area, $m^2/gm$ | 67 | 161 | 113 | 224 | 173 | 101 |

(1) TRA=Tetrabutylammonium.
(2) Glass-lined stainless steel autoclave.
(3) First scan analysis.
(4) 2-liter stainless steel pressure vessels.
(5) Calculated as 100% anhydrous solids.

TABLE 8

| Examples | 35 | 36 |
|---|---|---|
| Starting Materials, gms | | |
| A. Na Silicate (28.8% $SiO_2$, 8.9% $Na_2O$ 62% $H_2O$) | 74.6 | 484.9 |
| $H_2O$ | 53.0 | 344.5 |
| B. $FeNH_4(SO_4)_2 \cdot 12H_2O$ | 9.2 | 49.8 |
| $H_2SO_4$ | 2.1 | 13.65 |
| $H_2O$ | 105 | 682.5 |
| C. Tetrabutylammonium bromide | 20.5 | 133.25 |
| D. ZSM-11 Seeds (gms, solids) | | 2.0 |
| Starting Composition, moles | | |
| $(TBA)_2O$ (1) | 1.0 | 1.0 |
| $Na_2O$ | 1.51 | 1.50 |
| $Na_2SO_4$ | 1.86 | 1.86 |
| $Fe_2O_3$ | 0.30 | 0.30 |
| $SiO_2$ | 11.26 | 11.26 |
| $H_2O$ | 355 | 356 |
| Crystallization Conditions | | Stirred |
| Temp °F | 210 | 300 |
| Days | 104 | 5 |
| X-Ray Analysis | ZSM-11 75% | ZSM-11 115% |
| Product Composition, wt. % | 537°C (1000°F) | |
| N | — | 0.55 |
| Na | 2.98 | 2.27 |
| Fe | 5.2 | 5.2 |
| $SiO_2$ | 85.0 | 86.22 |
| Ash | 99.5 | 98.4 |
| $Al_2O_3$ | 0.16 | 0.27 |
| Production Composition, moles (2) | | |
| $R_2O$ | — | 1.37 |
| $Na_2O$ | 4.57 | 3.44 |
| $Fe_2O_3$ | 3.3 | 3.24 |
| $Al_2O_3$ | 0.11 | 0.18 |
| $SiO_2$ | 100 | 100 |
| $SiO_2/Al_2O_3$ | 909 | 555 |
| Adsorption wt. % | 537°C (1000°F) | 537°C (1000°F) |
| Cy-$C_6$ | 5.1 | 6.2 |
| n-$C_6$ | | 9.1 |
| $H_2O$ | 3.8 | 18.3 |
| Surface Area $m^2/gm$ | | 275 |
| Molar Ratio $SiO_2/Al_2O_3$ | 903 | 543 |

(1) TBA=Tetrabutylammonium.
(2) Calculated as 100% anhydrous solids.

TABLE 9

| Example | 37 |
|---|---|
| Starting Materials — gms | |
| A. $FeNH_4(SO_4)_2 \cdot 12H_2O$ | 59.8 |
| $H_2SO_4$ | 13.6 |
| $H_2O$ | 682.5 |
| B. Tetrabutylammonium bromide | 133.25 |
| C. $(ETO)_4Si$ Gel (17.0% $SiO_2$) | 821.47 |
| NaOH | 55.68 |
| $H_2O$ | 35.97 |
| D. ZSM-11 Seeds (gms, solids) | 2.0 |
| Starting Composition, moles | |
| $(TBA)_2O$ | 1.0 |
| $Na_2O$ | 1.51 |
| $Na_2SO_4$ | 1.86 |
| $Fe_2O_3$ | 0.30 |
| $SiO_2$ | 11.26 |
| $H_2O$ | 376 |
| Crystallization Conditions | Stirred |
| Temp °F | 300 |
| Days | 5 |
| X-Ray Analysis | ZSM-11<br>125% |
| Product Composition, wt. % | |
| N | 0.55 |
| Na | 1.4 |
| Fe | 4.3 |
| $SiO_2$ | 76.0 |
| Ash | 85.0 |
| $Al_2O_3$ | 370 ppm (parts per million) |
| Product Composition, moles (1) | |
| $R_2O$ | 1.55 |
| $Na_2O$ | 2.41 |
| $Fe_2O_3$ | 3.04 |
| $Al_2O_3$ | 0.03 |
| $SiO_2$ | 100 |
| Molar Ratio $SiO_2/Al_2O_3$ | 3.333 |
| Adsorption, wt. % | 537°C (1000°F) |
| Cyclohexane | 6.9 |
| n-Hexane | 10.4 |
| Water | 16.2 |
| Surface Area, $m^2/gm$ | 306 |

(1) Calculated as 100% anhydrous solids.

TABLE 10

| Examples | 45 | 46 | 47 |
|---|---|---|---|
| Starting material | material of Ex. 23 | material of Ex. 26 | material of Ex. 27 |
| Pre-Treat | T10-537°C (1000°F) | T10-537°C (1000°F) | T10-537°C (1000°F) |
| 10% $NH_4Cl$ Treat 87—90°C (190—195°F) | 4×1 hr | 3×1 hr<br>1×2 hrs | 3×1 hr<br>1×2 hrs |
| 10% $NH_4Cl$ Treat (160°F) 71°C | 16 hrs 48°C (120°F) | 16 hrs | 16 hrs |
| Composition, wt. % | | | |
| Na | 0.14 | 0.33 | 0.10 |
| Cr | 1.9 | 2.0 | 0.15 |
| $SiO_2$ | 92.9 | 91.3 | 95.8 |
| Ash | 98.1 | 98.1 | 100 |
| $Al_2O_3$ | 0.23 | 0.55 | 0.22 |
| Product Composition, moles (1) | | | |
| $R_2O$ | — | — | — |
| $Na_2O$ | 0.19 | 0.46 | 0.14 |
| $Cr_2O_3$ | 1.18 | 1.26 | 0.09 |
| $Fe_2O_3$ | — | — | — |
| $Al_2O_3$ | 0.15 | 0.35 | 0.14 |
| $SiO_2$ | 100 | 100 | 100 |
| Molar Ratio $SiO_2/Al_2O_3$ | 667 | 286 | 714 |
| $\alpha$-Test (1000°F) 537°C | | | |
| n-Hexane Conv. wt. % | | | |
| 5 min. | 7.7 | 17.1 | 0.7 |
| 25 min. | 5.4 | 14.4 | 0.5 |
| $\alpha$-Value 5 min. | 1.5 | 3.6 | 0.13 |
| 25 min. | 1.1 | 3.0 | 0.10 |
| Propylene Conv. wt. % (700°F) 371°C | 31.1 | 71.0 | |
| Selectivity | | | |
| $C_4^=$ | 19.7 | 31.7 | |
| $C_5^+$ | 75.2 | 64.0 | |

(1) Normalized to 100% solids.

TABLE 10 (contd.)

| Examples | 48 | 49 | 50 |
|---|---|---|---|
| Starting material | material of Ex. 31 | material of Ex. 35 | material of Ex. 36 |
| Pre-Treat | T10-537°C (1000°F) (85—87°C) (185—190°F) | T10-537°C (1000°F) | T10-537°C (1000°F) (85—87°C) (185—190°F) |
| 10% $NH_4Cl$ Treat 87—90°C (190—195°F) | 3×2 hrs | 3×1 hr 1×2 hrs | 1×1 hr 2×2 hrs |
| 10% $NH_4Cl$ Treat (160°F) 71°C | 16 hrs 62°C (145°F) | 18 hrs | 16 hrs 61°C (142°F) |
| Composition, wt. % | | | |
| Na | 0.20 | 0.22 | 0.34 |
| Cr | 0.79 | Fe 5.4 | Fe 5.1 |
| $SiO_2$ | 91.3 | 87.1 | 89.2 |
| Ash | 99.8 | 99.5 | 99.0 |
| $Al_2O_3$ | 0.36 | 0.18 | 0.24 |
| Product Composition, moles (1) | | | |
| $R_2O$ | — | — | — |
| $Na_2O$ | 0.28 | 0.33 | 0.49 |
| $Cr_2O_3$ | 0.09 | — | — |
| $Fe_2O_3$ | — | 3.34 | 3.07 |
| $Al_2O_3$ | 0.23 | 0.12 | 0.16 |
| $SiO_2$ | 100 | 100 | 100 |
| Molar Ratio $SiO_2/Al_2O_3$ | 435 | 833 | 625 |
| α-Test (1000°F) 537°C | | | |
| n-Hexane Conv. wt. % | | | |
| 5 min. | 0.3 | 1.8 | 8.7 |
| 25 min. | 0.2 | 0.7 | 3.8 |
| α-Value 5 min. | 0.06 | 0.34 | 1.73 |
| 25 min. | 0.04 | 0.13 | 0.75 |
| Propylene Conv. wt. % (700°F) 371°C | | | |
| Selectivity | | | |
| $C_4^=$ | | | |
| $C_5^+$ | | | |

(1) Normalized to 100% solids.

## Claims

1. Zeolite ZSM-11 having the composition, in terms of moles of oxides:

$$(0\text{—}10)\ M_{2/n}O:\ (0\text{—}0.5)\ Al_2O_3:\ 100\ SiO_2$$

in which M is at least one cation of valence n.

2. A zeolite according to claim 1, wherein c is at least 0.001.

3. A zeolite according to claim 1 or claim 2 in which c is no greater than 0.4.

4. A zeolite according to any preceding claim which has the composition, in terms of moles of oxides:

$$(0\text{—}10)\ M_{2/n}O:\ [(a)\ Cr_2O_3+(b)\ Fe_2O_3+(c)\ Al_2O_3]:\ 100\ SiO_2$$

in which (a) is from 0 to 4, (b) is from 0 to 5, (c) is 0.001 to 0.5, (a) and (b) cannot both be zero and when (a) or (b) is zero (b) or (a) respectively is greater than (c).

5. A zeolite according to claim 4 having the composition:

$$(0\text{—}3)\ R_2O:\ (0\text{—}8)\ M_{2/n}O:\ [(a)\ Cr_2O_3+(b)\ Fe_2O_3+(c)\ Al_2O_3]:\ 100\ SiO_2$$

in which $R_2O$ is the oxide form of an organic compound containing an element of Group 5A of the

Periodic Table which compound comprises an alkyl or aryl group having from 1 to 7 carbon atoms and M is an alkali or alkaline earth metal.

6. A zeolite according to any of claims 1 to 3 having the composition:

$$(>0—10)\ R_2O:\ (>0—10)\ M_{2/n}O:\ (0—0.5)\ Al_2O_3:\ 100\ SiO_2$$

in which $R_2O$ is the oxide form of an organic compound containing an element of Group 5A of the Periodic Table, said compound comprising an alkyl or aryl group having from 1 to 7 carbon atoms at least one of which is ethyl or butyl, and M is an alkali or alkaline earth metal.

7. A zeolite according to claim 5 or claim 6, wherein R is tetrabutylammonium and M is sodium.

8. A zeolite according to any of claims 1 to 4, wherein M is hydrogen, ammonium and/or rare earth.

9. A method for preparing zeolite ZSM-11 as claimed in claim 1 which comprises preparing a reaction mixture containing a source of an alkali metal oxide, an oxide of silicon, $R_2O$ and water and having a composition, in terms of mole ratios of oxides, falling within the following ranges:

$SiO_2/R_2O$=10 to 20
$M_2O/R_2O$=0.09 to 3.0
$H_2O/R_2O$=300 to 400

wherein $R_2O$ is the oxide form of an organic compound of an element of Group 5A of the Periodic Table, said organic compound comprising an alkyl or aryl group having between 1 and 7 carbon atoms, M is an alkali or alkaline earth metal, and maintaining the mixture at crystallization temperature until crystals of said zeolite are formed.

10. A method according to claim 9, wherein $R_2O$ comprises at least one ethyl or butyl radical.

11. A method according to claim 9 or claim 10, wherein the reaction mixture further comprises sources of chromic oxide and ferric oxide and its composition is further characterised by a value 0.2 to 1.0 for the mole ratio $(Cr_2O_3+Fe_2O_3)/R_2O$.

12. A method according to claim 11, wherein the reaction mixture has the composition:

$SiO_2/R_2O$=10 to 20
$(Na_2O+K_2O)/R_2O$=0.9 to 3.0
$(Cr_2O_3+Fe_2O_3)/R_2O$=0.2 to 0.4
$H_2O/R_2O$=300 to 400.

13. A method according to any of claims 9 to 12, wherein $R_2O$ is a quaternary compound.

14. A method according to any of claims 9 to 13, wherein R is tetrabutylammonium or tetrabutylphosphonium.

15. Use in converting an organic charge of a catalyst comprising zeolite ZSM-11 as claimed in any of claims 1 to 8.

**Patentansprüche**

1. Zeolith ZSM-11 der folgenden, als Mole Oxide ausgedrückten Zusammensetzung:

$$(0—10)\ M_{2/n}O:\ (0—0{,}5)\ Al_2O_3:\ 100\ SiO_2$$

worin M wenigstens ein Kation der Valenz n ist.

2. Zeolith nach Anspruch 1, bei dem c wenigstens 0,001 ist.

3. Zeolith nach Anspruch 1 oder 2, bei dem c nicht größer als 0,4 ist.

4. Zeolith nach einem beliebigen der vorangehenden Ansprüche, der ausgedrückt in Molen Oxide die Zusammensetzung aufweist:

$$(0—10)\ M_{2/n}O:\ [(a)\ Cr_2O_3+(b)\ Fe_2O_3+(c)\ Al_2O_3]:\ 100\ SiO_2$$

worin (a) von 0 bis 4 ist, (b) von 0 bis 5 ist, (c) von 0,001 bis 0,5 ist, (a) und (b) nicht beide 0 sein können und wenn (a) oder (b) 0 ist, (b) bzw. (a) größer als (c) sind.

5. Zeolith nach Anspruch 4 mit der Zusammensetzung:

$$(0—3)\ R_2O:\ (0—8)\ M_{2/n}O:\ [(a)\ Cr_2O_3+(b)\ Fe_2O_3+(c)\ Al_2O_3]:\ 100\ SiO_2,$$

wobei $R_2O$ die Oxidform einer organischen Verbindung, die ein Element der Gruppe 5A des Periodensystems der Elemente enthält, ist, wobei diese Verbindung eine Alkyl- oder Aryl-Gruppe mit von 1 bis 7 Kohlenstoffatomen enthält und wobei M ein Alkali- oder Erdalkali-Metall ist.

6. Zeolith nach einem beliebigen der Ansprüche 1 bis 3 mit der Zusammensetzung:

$$(>0—10)\ R_2O:\ (>0—10)\ M_{2/n}O:\ (0—0,5)\ Al_2O_3:\ 100\ SiO_2$$

wobei $R_2O$ die Oxidform einer organischen Verbindung, die ein Element der Gruppe 5A des Periodensystems der Elemente enthält, ist, wobei diese Verbindung eine Alkyl- oder Aryl-Gruppe mit von 1 bis 7 Kohlenstoffatomen enthält, wobei wenigstens eine davon Ethyl oder Butyl ist, und worin M ein Alkali- oder Erdalkali-Metall ist.

7. Zeolith nach Anspruch 5 oder 6, worin R Tetrabutylammonium ist und M Natrium ist.

8. Zeolith nach einem beliebigen der Ansprüche 1 bis 4, worin M Wasserstoff, Ammonium under/oder eine Seltene Erde ist.

9. Verfahren zur Herstellung des Zeoliths ZSM-11, wie er in Anspruch 1 beansprucht wird, das umfaßt: die Herstellung einer Reaktionsmischung, die eine Quelle für ein Alkalimetalloxid, ein Siliciumoxide, $R_2O$ und Wasser enthält und eine Zusammensetzung aufweist, ausgedrückt in Molverhältnissen der Oxide, die in die folgenden Bereiche fallen:

$$SiO_2/R_2O=10 \text{ bis } 20$$
$$M_2O/R_2O=0{,}09 \text{ bis } 3{,}0$$
$$H_2O/R_2O=300 \text{ bis } 400$$

worin $R_2O$ die Oxidform einer organischen Verbindung eines Elements der Gruppe 5A des Periodensystems der Elemente ist, wobei diese organische Verbindung eine Alkyl- oder Aryl-Gruppe mit zwischen 1 und 7 Kohlenstoffatomen aufweist, M ein Alkali- oder Erdalkali-Metall ist, und Halten der Mischung auf Kristallisationstemperatur, bis sich Kristalle dieses Zeolithen gebildet haben.

10. Verfahren nach Anspruch 9, bei dem $R_2O$ wenigstens einen Ethyl- oder Butyl-Rest enthält.

11. Verfahren nach Anspruch 9 oder 10, bei dem die Reaktionsmischung außerdem Quellen für Chrom(III)oxid und Eisen(III)oxid umfaßt und ihre Zusammensetzung außerdem durch einen Wert von 0,2 bis 1,0 für das Molverhältnis $(Cr_2O_3+Fe_2O_3)/R_2O$ gekennzeichnet ist.

12. Verfahren nach Anspruch 11, bei dem die Reaktionsmischung die folgende Zusammensetzung aufweist:

$$SiO_2/R_2O=10 \text{ bis } 20$$
$$(Na_2O+K_2O)/R_2O=0{,}9 \text{ bis } 3{,}0$$
$$(Cr_2O_3+Fe_2O_3)R_2O=0{,}2 \text{ bis } 0{,}4$$
$$H_2O/R_2O=300 \text{ bis } 400.$$

13. Verfahren nach einem beliebigen der Ansprüche 9 bis 12, bei dem $R_2O$ eine quaternäre Verbindung ist.

14. Verfahren nach einem beliebigen der Ansprüche 9 bis 13, bei dem R Tetrabutylammonium oder Tetrabutylphosphonium ist.

15. Verwendung eines Katalysators, der Zeolith ZSM-11, wie er in einem beliebigen der Ansprüche 1 bis 8 beansprucht wird, umfaßt, zur Umwandlung eines organischen Einsatzprodukts.

**Revendications**

1. Zéolite ZSM-11 présentant la composition exprimée en termes de moles d'oxydes:

$$(0—10)\ M_{2/n}O:\ (0—0,5)\ Al_2O_3:\ 100\ SiO_2$$

dans laquelle:

M est au moins un cation de valence n.

2. Une zéolite suivant la revendication 1, dans laquelle c est au moins égal à 0,001.

3. Une zéolite suivant la revendication 1 ou 2, dans laquelle c n'est pas supérieur à 0,4.

4. Une zéolite selon une quelconque des revendications précédentes qui présente la composition en termes de moles d'oxydes:

$$(0—10)\ M_{2/n}O:\ [(a)\ Cr_2O_3+(b)\ Fe_2O_3+(c)\ Al_2O_3]:\ 100\ SiO_2$$

dans laquelle:

(a) est compris entre 0 et 4,

(b) est compris entre 0 et 5,

(c) est compris entre 0,001 et 0,5,

(a) et (b) ne peuvent être simultanément égaux à 0 et lorsque (a) ou (b) sont égaux à 0, (b) ou (a) respectivement est supérieur à c.

5. Une zéolite selon la revendication 4 présentant la composition:

$$(0—3)\ R_2O:\ (0—8)\ M_{2/n}O:\ [(a)\ Cr_2O_3+(b)\ Fe_2O_3+(c)\ Al_2O_3]:\ 100\ SiO_2$$

dans laquelle:

$R_2O$ est la forme oxydée d'un composé organique contenant un élément du groupe 5A de la classification périodique lequel composé comprend un groupe alkyle ou aryle présentant de 1 à 7 atomes de carbone; et

M est un métal alcalin ou alcalino terreux.

6. Une zéolite selon une quelconque des revendications 1 à 3, présentant la composition:

$$(>0—10)\ R_2O:\ (>0—10)\ M_{2/n}O:\ (0—0{,}5)\ Al_2O_3:\ 100\ SiO_2$$

dans laquelle:

$R_2O$ est la forme oxydée d'un composé organique contenant un élément du groupe 5A de la classification périodique lequel composé comprenant un groupe alkyle ou aryle présentant de 1 à 7 atomes de carbone, dont au moins un est un radical éthyle ou butyle; et

M est un métal alcalin ou alcalino terreux.

7. Une zéolite selon la revendication 5 ou 6 dans laquelle R est le tétrabutylammonium est M est le sodium.

8. Une zéolite selon une quelconque des revendications 1 à 4, dans laquelle M est l'hydrogène, l'ammonium et/ou une terre rare.

9. Un procédé de préparation de zéolite ZSM-11 tel que revendiqué dans la revendication 1 qui consiste à préparer un mélange réactionnel contenant une source d'un oxyde métal alcalin, un oxyde de silicium, $R_2O$ et de l'eau, et présentant une composition, en termes de rapports molaires des oxydes, comprise dans les domaines suivants:

$$SiO_2/R_2O=10 \text{ à } 20$$
$$M_2O/R_2O=0{,}09 \text{ à } 3{,}0$$
$$H_2O/R_2O=300 \text{ à } 400$$

dans lequel:

$R_2O$ est la forme oxydée d'un composé organique d'un élément du groupe 5A de la classification périodique, ce composé organique comprenant un groupe alkyle ou aryle, ayant de 1 à 7 atomes de carbone;

M étant un métal alcalin ou alcalino terreux;

et le mélange étant maintenu à la température de cristallisation jusqu'à ce que les cristaux de ladite zéolite se soient formés.

10. Un procédé selon la revendication 9, dans lequel $R_2O$ comprend au moins un radical éthyle ou butyle.

11. Un procédé selon la revendication 9 ou la revendication 10, dans lequel le mélange réactionnel comprend, en outre, des sources d'oxydes chromiques et d'oxydes ferriques et sa composition est, de plus, caractérisée par une valeur du rapport molare $(Cr_2O_3+Fe_2O_3)R_2O$, compris entre 0,2 et 1,0.

12. Un procédé selon la revendication 11, dans lequel le mélange réactionnel présente la composition:

$$SiO_2/R_2O=10 \text{ à } 20$$
$$(Na_2O+K_2O)/R_2O=0{,}9 \text{ à } 3{,}0$$
$$(Cr_2O_3+Fe_2O_3)/R_2O=0{,}2 \text{ à } 0{,}4$$
$$H_2O/R_2O=300 \text{ à } 400.$$

13. Un procédé selon une quelconque des revendications 9 à 12, dans lequel $R_2O$ est un composé quaternaire.

14. Un procédé selon une quelconque des revendications 9 à 13, dans lequel R est le tétrabutylammonium ou le tétrabutylphosphonium.

15. Emploi, pour la conversion d'une charge organique, d'un catalyseur consistant et une zéolite ZSM-11 telle que revendiquée dans l'une quelconque des revendications 1 à 8.